(19) Europäisches Patentamt
European Patent Office
Office européen des brevets

(11) EP 3 463 360 B2

(12) NEW EUROPEAN PATENT SPECIFICATION
After opposition procedure

(45) Date of publication and mention
of the opposition decision:
**15.07.2026 Bulletin 2026/29**

(45) Mention of the grant of the patent:
**31.08.2022 Bulletin 2022/35**

(21) Application number: **17802848.6**

(22) Date of filing: **24.05.2017**

(51) International Patent Classification (IPC):
***A61K 31/46*** $^{(2006.01)}$ ***A61K 9/08*** $^{(2006.01)}$
***A61K 47/02*** $^{(2006.01)}$ ***A61K 47/10*** $^{(2017.01)}$
***A61K 47/12*** $^{(2006.01)}$ ***A61K 47/18*** $^{(2017.01)}$
***A61K 47/26*** $^{(2006.01)}$ ***A61K 47/38*** $^{(2006.01)}$

(52) Cooperative Patent Classification (CPC):
**A61K 9/0048; A61K 9/08; A61K 31/46;
A61K 47/02; A61K 47/10; A61K 47/12;
A61P 27/02; A61P 27/10**

(86) International application number:
**PCT/JP2017/019423**

(87) International publication number:
**WO 2017/204262 (30.11.2017 Gazette 2017/48)**

# (54) ATROPINE-CONTAINING AQUEOUS COMPOSITION

ATROPINHALTIGE WÄSSRIGE ZUSAMMENSETZUNG

COMPOSITION AQUEUSE CONTENANT DE L'ATROPINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.05.2016 SG 10201604200P**

(43) Date of publication of application:
**10.04.2019 Bulletin 2019/15**

(60) Divisional application:
**22189717.6 / 4 112 055**

(73) Proprietors:
- **Singapore Health Services Pte Ltd
Singapore 168582 (SG)**
- **Nanyang Technological University
Singapore 639798 (SG)**
- **Santen Pharmaceutical Co., Ltd.
Kita-ku
Osaka-shi
Osaka 530-8552 (JP)**

(72) Inventors:
- **TAN, Donald
168751 (SG)**
- **BEUERMAN, Roger
168751 (SG)**
- **ASADA, Hiroyuki
Ikoma-shi
Nara 630-0101 (JP)**
- **TAKAHASHI, Kyohei
Ikoma-shi
Nara 630-0101 (JP)**
- **SAKANAKA, Koji
Ikoma-shi
Nara 630-0101 (JP)**
- **MORIMOTO, Takashi
Ikoma-shi
Nara 630-0101 (JP)**
- **FUJISAWA, Toyomi
Ikoma-shi
Nara 630-0101 (JP)**

(74) Representative: **Müller-Boré & Partner
Patentanwälte PartG mbB
Friedenheimer Brücke 21
80639 München (DE)**

(56) References cited:

| | |
|---|---|
| WO-A1-02/096418 | WO-A1-2008/144065 |
| WO-A1-2012/161655 | WO-A1-2015/200361 |
| WO-A1-2018/209051 | JP-A- 2007 308 398 |
| JP-A- 2007 308 398 | JP-A- H01 203 320 |
| US-A1- 2007 254 914 | US-A1- 2016 009 705 |

- PO-CHIUNG FANG ET AL: "Prevention of Myopia Onset with 0.025% Atropine in Premyopic Children", JOURNAL OF OCULAR PHARMACOLOGY AND THERAPEUTICS., vol. 26, no. 4, 1 August 2010 (2010-08-01), US, pages 341 - 345, XP055535128, ISSN: 1080-7683, DOI: 10.1089/jop.2009.0135

## Description

The present invention relates to an aqueous composition that comprises atropine or a salt thereof (hereinafter also referred to simply as "atropine").

Myopia, a type of refractive error, is a condition of eyes where light coming into an eye from a distance is not focused on retina, but focused before retina, which causes the image of an object to appear blurred. It is known that myopia is caused by an ocular axial length (length from the cornea to the retina) that is longer than normal (axial myopia) or by excessively high refractive powers of the cornea or the crystalline lens (refractive myopia).

Atropine is known to have the property of preventing the elongation of an ocular axial length. For example, Patent Literature 1 discloses that a composition comprising less than 0.025% atropine inhibits or prevents myopia progression.

On the other hand, an atropine ophthalmic solution is used as a mydriatic, and also reduces accommodation. An atropine ophthalmic solution, when instilled into the eye, relaxes the pupillary sphincter muscle of the iris and thus induces mydriasis that causes glare, which persists for a period during which the action of the atropine ophthalmic solution is maintained, and also reduces accommodation of the crystalline lens to result in poor near-acuity. This can be a hindrance in performing daily activities. It would therefore be highly desirable that a medication for inhibiting or preventing myopia progression, should induce a lesser degree of mydriasis and a lesser loss of accommodation so as to enhance the quality of life (QOL).

[Patent Literature 1] WO 2012/161655 A1 US 2016/009705 A1 and WO 2015/200361 A1 describe aqueous ophthalmic formulations comprising Atropine (0.01-0.5 0.001-0.05 (wt %)), a buffer agent, the preservative benzalkonium chloride, a viscosity enhancing agent (e.g. hydroxypropyl methylcellulose) and show a pH = 4.2-7.9. The utilized diluents includes, but not limited to a phosphate buffered saline solution.

A purpose of the present invention is to find an aqueous composition comprising atropine which has a potent action for inhibiting the elongation of eye axial length and improving the refractive error. The important goal is to find an atropine-containing aqueous composition that induces a lesser degree of mydriasis and also a lesser loss of accommodation. And, another purpose of the present invention is to find an aqueous composition comprising atropine whose viscosity does not decrease with time and wherein atropine or a salt thereof is stable.

The present inventors have intensively studied to solve the aforementioned problem and consequently have found that an aqueous composition comprising 0.001 - 0.1 % (w/v) atropine or a salt thereof, a water-soluble polymer, and buffer (I), which is at a pH range of 6 or lower, wherein the buffer (I) is at least one selected from the group consisting of a phosphate buffer and an aminocarboxylate buffer, and wherein the water-soluble polymer is a cellulose derivative, and wherein the cellulose derivative is at least one selected from the group consisting of hydroxyethyl cellulose and hydroxypropyl methylcellulose surprisingly has a potent action for inhibiting the elongation of eye axial length and improving the refractive error without exacerbating the mydriatic action of atropine. Additionally, the present inventors have also found that the above aqueous composition, but which comprises no benzalkonium chloride or a limited amount of benzalkonium chloride, has a lower mydriatic action. Furthermore, the present inventors have also found that, in an aqueous composition comprising atropine or a salt thereof and a water-soluble polymer which is at a pH range of 6 or lower, the addition of a nonionic tonicity agent can make it possible to inhibit the debasement over time of the viscosity given by the water-soluble polymer and additionally maintain the stability of atropine or a salt thereof. The aqueous composition of the present invention is expected to inhibit or prevent the progression of myopia and lead to a lesser degree of mydriasis, and lesser loss of accommodation so as to be optimal in terms of quality of life.

That is, the present invention relates to an aqueous composition as characterized in the claims.

As is apparent from the test results which will be described later, it has been shown that an aqueous composition comprising 0.001 - 0.1 % (w/v) atropine or a salt thereof, a water-soluble polymer, and a buffer (I), which is at a pH range of 6 or lower, wherein the buffer (I) is at least one selected from the group consisting of a phosphate buffer and an aminocarboxylate buffer, wherein the water-soluble polymer is a cellulose derivative, and wherein the cellulose derivative is at least one selected from the group consisting of hydroxyethyl cellulose and hydroxypropyl methylcellulose, has a potent action for inhibiting the elongation of eye axial length and improving the refractive error without exacerbating the mydriatic action of atropine. Additionally, it has been also shown that the above aqueous composition, but which comprises no benzalkonium chloride or a limited amount of benzalkonium chloride, has a lower mydriatic action. Furthermore, it has been also shown that, in an aqueous composition comprising atropine or a salt thereof and a water-soluble polymer which is at a pH range of 6 or lower, the addition of a nonionic tonicity agent can make it possible to inhibit the debasement over time of the viscosity given by the water-soluble polymer and additionally maintain the stability of atropine or a salt thereof. The present aqueous composition is therefore expected to inhibit or prevent the progression of myopia and lead to a lesser degree of mydriasis, and lesser loss of accommodation so as to be optimal in terms of quality of life. A further advantage associated with the compositions of the present invention, such as compositions that further comprise a tonicity agent, is that the compositions may retain their initial viscosity (or a substantial proportion thereof) over an extended period of time.

Fig. 1 shows the results of the viscosity determination test in Test 5.

Fig. 2 shows the results of the stability test in Test 5.
Fig. 3 shows the results of Examples 23 to 25 in the viscosity determination test in Test 6.
Fig. 4 shows the results of Examples 26 to 28 in the viscosity determination test in Test 6.

The present aqueous composition comprises "atropine or a salt thereof," which serves as an active ingredient.

In the present invention, the term "atropine or a salt thereof' also includes (i) a hydrate of atropine or a salt thereof, (ii) an organic solvate of atropine or a salt thereof, and (iii) a combination of the hydrate and the organic solvate.

The salt of atropine includes atropine sulfate or a hydrate thereof, and is preferably an atropine sulfate hydrate.

Atropine sulfate hydrate is a compound represented by the following structural formula:

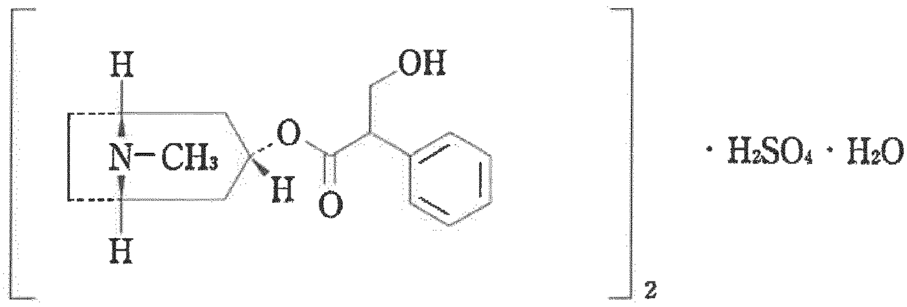

In a case where the atropine or the salt thereof includes a crystal polymorph and a group of crystal polymorphs (crystal polymorph system), those crystal polymorph and group of crystal polymorphs (crystal polymorph system) are also encompassed by the scope of the present invention. Here, the group of crystal polymorphs (crystal polymorph system) means not only individual crystal forms obtained at respective stages where crystals transform into various forms depending on conditions and states during the manufacture, crystallization, storage, and the like of the crystals, but also a mixture of the crystal forms obtained at two or more of the stages.

Atropine or a salt thereof can be manufactured in accordance with a method commonly employed in the field of organic synthetic chemistry or can alternatively be a commercially available product. For example, atropine sulfate hydrate can be a commercially available product from Tokyo Chemical Industry Co., Ltd. (product code: A0550).

In the present invention, the concentration of atropine or a salt thereof is 0.001 to 0.1 % (w/v), preferably 0.001 to 0.05 % (w/v), more preferably 0.001 to 0.025 % (w/v), and particularly preferably 0.001 to 0.01 % (w/v). More specifically, the concentration is preferably 0.0010 % (w/v), 0.0015 % (w/v), 0.0020 % (w/v), 0.0025 % (w/v), 0.0030 % (w/v), 0.0035 % (w/v), 0.0040 % (w/v), 0.0045 % (w/v), 0.0050 % (w/v), 0.0055 % (w/v), 0.0060 % (w/v), 0.0065 % (w/v), 0.0070 % (w/v), 0.0075 % (w/v), 0.0080 % (w/v), 0.0085 % (w/v), 0.0090 % (w/v), 0.0095 % (w/v), or 0.010 % (w/v).

In the present invention, the term "aqueous composition" means a composition containing water that serves as a solvent.

In the present invention, the "water-soluble polymer" includes hydroxyethyl cellulose and hydroxypropyl methylcellulose.

The present aqueous composition can comprise one or more types of water-soluble polymers.

In the present invention, the concentration of the water-soluble polymer in the present aqueous composition is set to a value by adjusting the content of a water-soluble polymer as appropriate to reflect the influence of the water-soluble polymer on a medicinal substance (active ingredient), other additive(s), pH, osmotic pressure, and/ or viscosity. However, the concentration of the water-soluble polymer in the present aqueous composition is preferably 0.01 to 5 % (w/v), more preferably 0.1 to 2 % (w/v), still more preferably 0.1 to 1 % (w/v), particularly preferably 0.1 to 0.6 %.

In the present invention, in a case where the water-soluble polymer is hydroxyethyl cellulose, the concentration of the hydroxyethyl cellulose is preferably 0.1 to 1.0 % (w/v), and more preferably 0.1 to 0.6 % (w/v).

In the present invention, in a case where the water-soluble polymer is hydroxypropyl methylcellulose, the concentration of the hydroxypropyl methylcellulose is preferably 0.1 to 1.0 % (w/v), and more preferably 0.1 to 0.6 % (w/v).

In the present invention, generally, the term "buffer" should not be limited as long as it is pharmaceutically acceptable ones, which includes, a phosphate buffer, and an aminocarboxylate buffer. The aminocarboxylate buffer includes, for example, an aspartate buffer, a glutamate buffer, and epsilon-aminocaproic acid. These buffers may be used as a single ingredient or as a combination of any two or more ingredients.

In the present invention, the concentration of the buffer in the present aqueous composition is set to a value by adjusting the content of the buffer as appropriate to reflect the influence of the buffer on a medicinal substance (active ingredient), other additive(s), pH, osmotic pressure, and/or viscosity. However, the concentration of the buffer in the present aqueous composition is preferably 0.001 to 10 % (w/v), more preferably 0.01 to 5 % (w/v), still more preferably 0.01 to 3 % (w/v), still much more preferably 0.01 to 1 % (w/v), particularly preferably 0.01 to 0.5 % (w/v), more particularly

preferably 0.01 to 0.1 % (w/v), wherein the weight of the buffer is that of a buffering agent as its material.

In the present invention, the phosphate buffer can be derived from any pharmaceutically acceptable phosphate buffering agent. Non-limiting examples of such a phosphate buffering agent include: phosphoric acid; phosphates such as alkali metal phosphates and alkaline earth metal phosphates; and hydrates thereof. More specifically, the phosphate buffering agent includes dibasic sodium phosphate hydrate (referred to as "dibasic sodium phosphate" or "sodium phosphate"), sodium dihydrogen phosphate (referred to as "monosodium phosphate"), sodium dihydrogen phosphate monohydrate (referred to as "monosodium phosphate"), sodium dihydrogen phosphate dihydrate (referred to as "monosodium phosphate"), potassium dihydrogen phosphate (referred to as "monopotassium phosphate"), sodium monohydrogen phosphate heptahydrate, trisodium phosphate, dipotassium phosphate, and the like.

In the present invention, the concentration of the phosphate buffer in the present aqueous composition is set to a value by adjusting the content of the phosphate buffer as appropriate to reflect the influence of the phosphate buffer on a medicinal substance (active ingredient), other additive(s), pH, osmotic pressure, and/or viscosity. However, the concentration of the phosphate buffer in the present aqueous composition is preferably 0.01 to 1.0 % (w/v), more preferably 0.05 to 1.0 % (w/v), and still more preferably 0.05 to 0.5 % (w/v), wherein the weight of the phosphate buffer is that of a phosphate buffering agent as its material.

In the present invention, the "citrate buffer" can be derived from a citrate buffering agent which should not be limited as long as it is pharmaceutically acceptable ones, which includes, for example, citric acid; citrates such as alkali metal citrates and alkaline earth metal citrates; and hydrates thereof. More specifically, the citrate buffer includes citric acid hydrate, sodium citrate, sodium citrate hydrate, potassium citrate, calcium citrate, sodium dihydrogen citrate, and disodium citrate.

In the present invention, the concentration of the citrate buffer in the present aqueous composition is set to a value by adjusting the content of citrate buffer as appropriate to reflect the influence of the citrate buffer on a medicinal substance (active ingredient), other additive(s), pH, osmotic pressure, and/or viscosity. However, the concentration of the citrate buffer in the present aqueous composition is preferably 0.001 to 1.0 % (w/v), more preferably 0.005 to 0.5 % (w/v), still more preferably 0.01 to 0.1 % (w/v), still much more preferably 0.01 to 0.05 % (w/v) and particularly preferably 0.02 to 0.04 % (w/v), wherein the weight of the citrate buffer is that of a citrate buffering agent as its material.

In the present invention, the "borate buffer" can be derived from a borate buffering agent which includes, for example, boric acid or a salt thereof, and borax. More specifically, the borate buffering agent includes boric acid, sodium borate, potassium borate, potassium tetraborate, potassium metaborate, ammonium borate, and borax. The "carbonate buffer" can be derived from a carbonate buffering agent which includes, for example, carbonic acid or a salt thereof. More specifically, the carbonate buffering agent includes carbonic acid, sodium bicarbonate, sodium carbonate, ammonium carbonate, potassium carbonate, calcium carbonate, potassium bicarbonate, and magnesium carbonate. The "acetate buffer" can be derived from an acetate buffering agent which includes, for example, acetic acid or a salt thereof. More specifically, the acetate buffering agent includes acetic acid, ammonium acetate, potassium acetate, calcium acetate, and sodium acetate. The "tartrate buffer" can be derived from a tartrate buffering agent which includes, for example, tartaric acid or a salt thereof. More specifically, the tartrate buffering agent includes sodium tartrate and potassium tartrate. The "aspartate buffer" can be derived from an aspartate buffering agent which includes, for example, aspartic acid or a salt thereof. More specifically, the aspartate buffering agent includes sodium aspartate and magnesium aspartate. The "glutamate buffer" can be derived from a glutamate buffering agent which includes, for example, glutamic acid or a salt thereof. More specifically, the glutamate buffering agent includes sodium glutamate and potassium glutamate.

The aqueous composition of the present invention may comprise buffer (I) as sole buffers, or buffer (I) and buffer (II) as sole buffers. And, the aqueous composition of the present invention may also comprise further different buffers besides buffer (I) and buffer (II).

In the present invention, the term "buffer (I)" is at least one selected from the group consisting of a phosphate buffer and an aminocarboxylate buffer. The definition of each buffer and the preferred range of each concentration are as explained in the above section of "buffer".

In the present invention, the term "buffer (II)" is a citrate buffer. The definition of the citrate buffer and the preferred range of the concentration are as explained in the above section of "buffer".

The viscosity of the present aqueous composition is adjusted to fall within preferably a range of 3 to 500 mPa·s, and more preferably a range of 6 to 70 mPa·s, when measured by an E-type viscometer (25°C; shear rate of 50 $s^{-1}$).

The present aqueous composition may further comprise a tonicity agent. The tonicity agent used in the present invention can be any pharmaceutically acceptable tonicity agent. Non-limiting examples of such a tonicity agent include nonionic tonicity agents such as glycerin, mannitol, propylene glycol, polyethylene glycol, glucose, sorbitol, xylitol, and trehalose. In the present invention, a nonionic tonicity agent is preferable as the tonicity agent. As the nonionic tonicity agent, glycerin, mannitol, propylene glycol, polyethylene glycol, glucose, sorbitol, xylitol, and trehalose are preferable, glycerin and mannitol are more preferable, and glycerin is particularly preferable.

As the tonicity agent used in the present invention, the tonicity agents listed above may be used singly or in combination of two or more.

In the present invention, the concentration of the tonicity agent in the present aqueous composition is set to a value by adjusting the content of a tonicity agent as appropriate to reflect the influence of the tonicity agent on a medicinal substance (active ingredient), other additive(s), pH, osmotic pressure, and/or viscosity. However, the concentration of the tonicity agent in the present aqueous composition is preferably 0.01 to 10 % (w/v), more preferably 0.05 to 5 % (w/v), still more preferably 0.1 to 5 % (w/v), still much more preferably 0.5 to 5 % (w/v), and particularly preferably 1 to 5 % (w/v).

In the present invention, in a case where the tonicity agent is glycerin, the concentration of glycerin is preferably 0.1 to 5.0 % (w/v), more preferably 0.1 to 3.0 % (w/v), still more preferably 0.5 to 3.0 % (w/v) and particularly preferably 1.0 to 3.0 % (w/v).

The present aqueous composition can comprise a pharmaceutically acceptable additive(s) as needed. The additive(s) can be mixed with other ingredients of the present aqueous composition by a widely used technique. The additive(s) can be selected from, for example, surfactants such as polyoxyethylene sorbitan monooleate, polyoxyl 40 stearate, and polyoxyethylene hydrogenated castor oil; stabilizers such as disodium edetate; preservatives such as benzalkonium chloride and boric acid; pH adjusting agents such as hydrochloric acid and sodium hydroxide; and the like as needed.

In general, benzalkonium chloride is used as a preservative. In the present invention, it suggests that an aqueous composition of atropine which comprises no benzalkonium chloride has lower mydriatic action than another aqueous composition of atropine which comprises benzalkonium chloride, as shown later. Thus, it is preferable that the present aqueous composition comprises no benzalkonium chloride or a limited amount of benzalkonium chloride. The "limited amount" used herein means an amount of benzalkonium chloride used in the present aqueous composition of atropine which does not exacerbate the mydriatic action. Specifically, the concentration of benzalkonium chloride is preferably less than 100 ppm, more preferably less than 50 ppm, and even more preferably the present aqueous composition comprises substantially no benzalkonium chloride.

The term "unit-dose container" used herein means an eyedrop container in which a cap is tightly attached to the bottle mouth with fusion, which is opened by breaking the fused part between the cap and the bottle mouth when it is used. The unit-dose container may contain just one dose of the aqueous composition for one shot, or more doses thereof used several times in one day.

The term "multiple-dose container" used herein means an eyedrop container equipped with a bottle body and a cap which can be fixed to the bottle body, said cap can be freely opened and closed. The multiple-dose container generally contains plural doses of the eyedrop liquid for using for a certain period.

The aqueous composition of the present invention can be contained in a unit-dose container or a multiple-dose container. Unless the present aqueous composition substantially comprises a preservative such as benzalkonium chloride, a unit-dose container is preferable.

The pH of the present aqueous composition is not limited to a specific value, provided that it falls within a medicinally acceptable range of 6 or lower. However, the pH of the present aqueous composition is preferably in a range of 4 to 6, more preferably in a range of 4 to 5, and particularly preferably in the neighborhood of 4 or 5. More specifically, for example, pH 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, and 6.0 are preferable, and pH 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, and 5.4 are more preferable.

Further, the osmotic pressure of the aqueous composition in the present invention is not limited to a specific value, provided that it falls within a range acceptable to a living body. The osmotic pressure of the aqueous composition in the present invention is, for example, 100 to 1000 mOsm, preferably 200 to 500 mOsm, and more preferably 250 to 350 mOsm. In general, the osmotic pressure of an aqueous composition is more than a little affected by the amounts of medicinal substance and additive in the aqueous composition. In the present invention, the osmotic pressure can be adjusted to fall within the above-described ranges by appropriately adjusting the amounts of those substances that can affect the osmotic pressure. It should be noted that the osmotic pressure of the aqueous composition in the present invention can be measured by a common method. For example, the osmotic pressure of the aqueous composition in the present invention can be measured in accordance with the method described in the "Osmometry (Osmolarity Determination)" section of the Japanese Pharmacopoeia, 15th Revised Edition.

Examples of a dosage form of the present aqueous composition include an eyedrop or an ophthalmic aqueous solution.

The dosage and administration of the aqueous composition administered in the present invention are not limited as long as it can sufficiently provide a desired efficacy, which can be administered in eyedrops, preferably at a frequency of 1 - 5 times a day in an amount of 1 - 3 drops each time, more preferably at a frequency of 2 - 4 times a day in an amount of 1 - 2 drops each time, and the most preferably once a day, before bedtime in an amount of 1 drop.

The present aqueous composition is preferably used to inhibit or prevent the progression of myopia, to prevent myopia, and/or to treat myopia, and is more preferably used to inhibit or prevent the progression of childhood myopia.

The term "inhibit or prevent the progression of myopia" used herein may mean slowing myopia progression or reducing myopia progression. The term "prevent myopia" used herein may mean preventing the onset of myopia or

delaying the onset of myopia.

**Examples**

The test results and preparation examples shown below are given for better understanding of the present invention, but the scope of the present invention should not be limited thereto.
The meanings of abbreviates are as follows.

BAK: Benzalkonium chloride
CVP: Carboxyvinyl polymer
HEC: Hydroxyethyl cellulose
HPMC: Hydroxypropyl methylcellulose

Test 1

Some aqueous compositions were evaluated in terms of their mydriatic action.

(Sample Preparation Method)

(Example 1)

An aqueous composition in Example 1 was prepared in accordance with the formulation shown in Table 1. Specifically, 0.01 g of atropine sulfate hydrate, 0.32 g of hydroxyethyl cellulose, 0.1 g of sodium dihydrogen phosphate, and 2.4 g of concentrated glycerin were dissolved in purified water. To the solution thus obtained were added hydrochloric acid and sodium hydroxide as appropriate, so that the solution was adjusted to pH 5 and brought to a total volume of 100 ml.

(Examples 2 and 3 and Comparative Examples 1 to 3)

Aqueous compositions in Examples 2 and 3 and in Comparative Examples 1 to 3 were prepared as in Example 1 in accordance with the formulation shown in Table 1.

Table 1 (Unit: g/100 ml)

| | Com. Ex. 1 | Com. Ex. 2 | Com. Ex. 3 | Ex. 1 | Ex. 2 | Ex.3 |
|---|---|---|---|---|---|---|
| Atropine sulfate hydrate | 0.01 | 0.01 | 0.01 | 0.01 | 0.004 | 0.01 |
| Hydroxyethyl cellulose | 0.32 | 0.32 | - | 0.32 | 0.32 | - |
| Sodium dihydrogen phosphate | 0.1 | - | - | 0.1 | 0.1 | 0.1 |
| Sodium citrate hydrate | - | 0.1 | - | - | - | - |
| Concentrated glycerin | 2.4 | 2.4 | 2.4 | 2.4 | 2.4 | 2.4 |
| pH adjusting agent | q.s. | q.s. | q.s. | q.s. | q.s. | q.s. |
| pH | 7 | 5 | 5 | 5 | 5 | 5 |
| Viscosity (mPa·s) | 31.77 | 30.42 | * | 31.30 | 33.94 | * |

*: unmeasured

(Test Method)

A single dose of each aqueous composition (50 μl in volume) was instilled into one eye of a rabbit (four eyes from four rabbits or six eyes from six rabbits for each aqueous composition). Images of pupils of the rabbits before the instillation and 1 hour after the instillation were captured by optical coherence tomography (OCT) and were then analyzed by image analysis software to calculate pupil areas of the rabbits and a mydriasis rate. The mydriasis rate was calculated by the following equation:

$$\text{Mydriasis rate (\%)} = ((b - a)/a) \times 100,$$

where *a* is a mean value ($mm^2$) of the pupil area before the instillation in each of Comparative Examples 1 to 3 and Examples 1 to 3, *a* is 16.8 ($mm^2$) and *b* is a value of the pupil area 1 hour after the instillation.

(Test Results)

The results in Examples 1 to 3 and in Comparative Examples 1 to 3 are shown in Table 2. In Table 2, each value is a mean value of data from the four or six cases.
The mydriatic action of each aqueous composition was evaluated under the following criteria.

A: in case that the pupil area is less than 30.0 $mm^2$ one hour after the instillation.
B: in case that the pupil area is 30.0 $mm^2$ to less than 35.0 $mm^2$ one hour after the instillation.
C: in case that the pupil area is 35.0 $mm^2$ to less than 40.0 $mm^2$ one hour after the instillation.
D: in case that the pupil area is 40.0 $mm^2$ or more one hour after the instillation.

Table 2

| | Value of pupil area 1 hour after the instillation ($mm^2$) | Mydriasis rate (%) | Evaluation |
|---|---|---|---|
| Comparative Example 1 | 40.8 | 143 | D |
| Comparative Example 2 | 40.1 | 139 | D |
| Comparative Example 3 | 43.4 | 158 | D |
| Example 1 | 35.3 | 110 | C |
| Example 2 | 31.9 | 90 | B |
| Example 3 | 35.1 | 109 | c |

(Discussion)

As is apparent from Table 2, it has been shown that an aqueous composition (i) comprising atropine or a salt thereof, (ii) having pH in a range of 6 or less, and (iii) further comprising a phosphate buffer, induces lesser degree of mydriasis than a composition comprising no phosphate buffer.
Test 2 Some aqueous compositions of the present invention were evaluated in terms of their mydriatic action.

(Sample Preparation Method)

(Examples 4 to 11) Aqueous compositions in Examples 4 to 11 were prepared as in Example 1 in accordance with the formulation shown in Table 3. Among those, Examples 5, 9, and 11 are not in accordance with the present invention.

(Test Method)

A single dose of each aqueous composition (50 μl in volume) was instilled into one eye of a rabbit (four eyes from four rabbits or six eyes from six rabbits for each aqueous composition). Images of pupils of the rabbits 1 hour after the instillation were captured by optical coherence tomography (OCT) and were then analyzed by image analysis software to calculate pupil areas of the rabbits.

(Test Results)

The results in Examples 4 to 11 are shown in Table 3. In Table 3, each value is a mean value of data from the four or six cases.
The mydriatic action of each aqueous composition was evaluated under the following criteria.

A: in case that the pupil area is less than 30.0 $mm^2$ one hour after the instillation.
B: in case that the pupil area is 30.0 $mm^2$ to less than 35.0 $mm^2$ one hour after the instillation.
C: in case that the pupil area is 35.0 $mm^2$ to less than 40.0 $mm^2$ one hour after the instillation.
D: in case that the pupil area is 40.0 $mm^2$ or more one hour after the instillation.

| Table 3 (Unit: g/100 ml) | | | | | | | | |
|---|---|---|---|---|---|---|---|---|
| Example | 4 | 5 | 6 | 7 | 8 | 9 | 10 | 11 |
| Atropine sulfate hydrate | 0.01 | 0.01 | 0.01 | 0.01 | 0.01 | 0.01 | 0.01 | 0.01 |
| Hydroxyethyl cellulose | 0.32 | 0.32 | 0.32 | 0.45 | 0.45 | 0.32 | 0.32 | 0.32 |
| Sodium dihydrogen phosphate | 0.1 | - | 0.1 | 0.1 | 0.1 | - | - | - |
| Sodium citrate hydrate | - | 0.02 | 0.02 | 0.02 | 0.04 | 0.04 | - | - |
| Epsilon-Aminocaproic acid | - | - | - | - | - | - | 0.04 | - |
| Acetic acid | - | - | - | - | - | - | - | 0.04 |
| Concentrated glycerin | 2.4 | 2.4 | 2.4 | 2.4 | 2.4 | 2.4 | 2.4 | 2.4 |
| pH adjusting agent | q.s. | q.s. | q.s. | q.s. | q.s. | q.s. | q.s. | q.s. |
| pH | 4.3 | 4.3 | 4.3 | 4.3 | 4.3 | 4.3 | 4.3 | 4.3 |
| Value of pupil area 1 hour after the instillation ($mm^2$) | 30.8 | 35.4 | 24.1 | 27.1 | 28.0 | 33.4 | 30.4 | 39.0 |
| Evaluation | B | C | A | A | A | B | B | C |

(Discussion)

When the aqueous composition comprised a phosphate buffer as buffer (I), the mydriatic action was low (Example 4). In addition, when the aqueous composition further comprised a citrate buffer as buffer (II) besides a phosphate buffer, the mydriatic action was lower (Examples 6 to 8).

Test 3

Some aqueous compositions of the present invention were evaluated in terms of their mydriatic action.

(Sample Preparation Method)

(Examples 12 to 14)

Aqueous compositions in Examples 12 to 14 were prepared as in Example 1 in accordance with the formulation shown in Table 4.

(Test Method)

A single dose of each aqueous composition (50 μl in volume) was instilled into one eye of a rabbit (four eyes from four rabbits for each aqueous composition). Images of pupils of the rabbits 1 hour after the instillation were captured by optical coherence tomography (OCT) and were then analyzed by image analysis software to calculate pupil areas of the rabbits.

(Test Results)

The results in Examples 12 to 14 are shown in Table 4. In Table 4, each value is a mean value of data from the four cases.

The mydriatic action of each aqueous composition was evaluated under the following criteria.

A: in case that the pupillary area one hour after eyedropping is less than 30.0 $mm^2$.
B: in case that the pupillary area one hour after eyedropping is 30.0 $mm^2$ to less than 35.0 $mm^2$.
C: in case that the pupillary area one hour after eyedropping is 35.0 $mm^2$ to less than 40.0 $mm^2$.
D: in case that the pupillary area one hour after eyedropping is 40.0 $mm^2$ or more.

| Table 4 (Unit: g/100 ml) | | | |
|---|---|---|---|
| Example | 12 | 13 | 14 |
| Atropine sulfate hydrate | 0.005 | 0.005 | 0.005 |
| Hydroxyethyl cellulose | 0.45 | 0.45 | 0.45 |
| Sodium dihydrogen phosphate | 0.1 | 0.1 | 0.1 |
| Sodium citrate hydrate | - | 0.03 | 0.04 |
| Concentrated glycerin | 2.4 | 2.4 | 2.4 |
| pH adjusting agent | q.s. | q.s. | q.s. |
| - pH | 4.3 | 4.3 | 4.3 |
| Value of pupil area 1 hour after the instillation ($mm^2$) | 25.0 | 24.2 | 20.8 |
| Evaluation | A | A | A |

(Discussion)

Even though the aqueous composition comprised 0.005 % (w/v) atropine, the mydriatic action was low when the aqueous composition comprised a phosphate buffer as buffer (I) (Example 12). In addition, when the aqueous composition further comprised a citrate buffer as buffer (II) besides a phosphate buffer, the mydriatic action was lower (Examples 13 and 14).

Test 4

The effect of benzalkonium chloride which is generally used as a preservative to the mydriatic action of the present aqueous composition was studied.

(Sample Preparation Method)

(Examples 15 to 17)

Aqueous compositions in Examples 15 to 17 were prepared as in Example 1 in accordance with the formulation shown in Table 5.

(Test Method)

A single dose of each aqueous composition (50 µl in volume) was instilled into one eye of a rabbit (four eyes from four rabbits for each aqueous composition). Images of pupils of the rabbits 1 hour after the instillation were captured by optical coherence tomography (OCT) and were then analyzed by image analysis software to calculate pupil areas of the rabbits.

(Test Results)

The results in Examples 15 to 17 are shown in Table 5. In Table 5, each value is a mean value of data from the four cases.

The mydriatic action of each aqueous composition was evaluated under the following criteria.

A: in case that the pupil area is less than 30.0 $mm^2$ one hour after the instillation.
B: in case that the pupil area is 30.0 $mm^2$ to less than 35.0 $mm^2$ one hour after the instillation.
C: in case that the pupil area is 35.0 $mm^2$ to less than 40.0 $mm^2$ one hour after the instillation.
D: in case that the pupil area is 40.0 $mm^2$ to less than 45.0 $mm^2$ one hour after the instillation.
E: in case that the pupil area is 45.0 $mm^2$ or more one hour after the instillation.

| Table 5 (Unit: g/100 ml) | | | |
|---|---|---|---|
| Example | 15 | 16 | 17 |
| Atropine sulfate hydrate | 0.01 | 0.01 | 0.01 |
| Hydroxyethyl cellulose | 0.53 | 0.53 | 0.53 |
| Sodium dihydrogen phosphate | 0.1 | 0.1 | 0.1 |
| Concentrated glycerin | 2.4 | 2.4 | 2.4 |
| BAK (Unit: ppm) | - | 50 | 100 |
| pH adjusting agent | q.s. | q.s. | q.s. |
| pH | 5 | 5 | 5 |
| Value of pupil area 1 hour after the instillation ($mm^2$) | 35.1 | 44.8 | 45.1 |
| Evaluation | c | D | E |

(Discussion)

As is clear from Table 5, the aqueous composition comprising no benzalkonium chloride (Example 15) exhibited lower mydriatic action than the aqueous composition comprising benzalkonium chloride (Examples 16 and 17).

Test 5

(Viscosity Determination Test 1 and Stability Test 1)

The effects of a tonicity agent to the viscosity of an aqueous composition comprising atropine and a water-soluble polymer and the stability of atropine therein were studied.

(Sample Preparation Method)

(Examples 18 to 22)

Aqueous compositions in Examples 18 to 22 were prepared as in Example 1 in accordance with the formulation shown in Table 6. Each prepared sample (5 mL) was put into a polyethylene eyedrop container, an inside plug was attached to the bottle mouth, and the container was sealed-up with a cap. The containers were stored under dark at 60°C for 4 weeks.

| Table 6 (Unit: g/100 ml) | | | | | |
|---|---|---|---|---|---|
| Example | 18 | 19 | 20 | 21 | 22 |
| Atropine sulfate hydrate | 0.01 | 0.01 | 0.01 | 0.01 | 0.01 |
| Hydroxyethyl cellulose | 0.45 | 0.45 | 0.45 | 0.45 | 0.45 |
| Sodium dihydrogen phosphate | 0.1 | 0.1 | 0.1 | 0.1 | 0.1 |
| Sodium chloride | - | 0.9 | - | - | - |
| Boric acid | - | - | 1.9 | - | - |
| Concentrated glycerin | - | - | - | 2.4 | - |
| Mannitol | - | - | - | - | 5 |
| pH adjusting agent | q.s. | q.s. | q.s. | q.s. | q.s. |
| pH | 5 | 5 | 5 | 5 | 5 |

(Test Method)

(1) Viscosity Determination Test 1

According to "Method II, Viscosity measurement by rotational viscometer" in the Japanese Pharmacopoeia 16th edition, each viscosity of the freshly-prepared aqueous compositions and the aqueous compositions stored for 1, 2 and 4 weeks after the productions was measured with a cone-flat plate-type rotational viscometer. The measuring conditions are shown below.

- Instrument: Rotary rheometer (Kinexus pro+)
- Rotating speed (S-1): 50/sec
- Measurement temperature: 25°C

(2) Stability Test 1

Tropic acid can be formed in the decomposition of atropine. In order to evaluate the stability of atropine in the present test, the content of tropic acid in the freshly-prepared aqueous compositions and the aqueous compositions stored for 1, 2 and 4 weeks after the productions was determined by high-performance liquid chromatography.

(Test Results)

The results of Viscosity Determination Test 1 are shown in Figure 1. The results of Stability Test 1 are shown in Figure 2.

(Discussion)

(1) Viscosity Determination Test 1

As shown in Figure 1, each viscosity of the aqueous composition comprising atropine and hydroxyethyl cellulose, but no tonicity agent (Example 18 which corresponds to "without" in Figure 1), the aqueous composition further comprising sodium chloride as a tonicity agent (Example 19 which corresponds to "NaCl" in Figure 1), and the aqueous composition further comprising boric acid as a tonicity agent (Example 20 which corresponds to "Boric acid" in Figure 1), decreased over time. On the other hand, each viscosity of the aqueous composition comprising atropine and hydroxyethyl cellulose, and further comprising glycerin as a tonicity agent (Example 21 which corresponds to "Glycerin" in Figure 1) and the aqueous composition further comprising mannitol as a tonicity agent (Example 22 which corresponds to "Mannitol" in Figure 1) was maintained, i.e., the decrease of viscosity over time was inhibited.

(2) Stability Test 1

As shown in Figure 2, more tropic acid was produced from the aqueous composition comprising atropine and hydroxyethyl cellulose, and further comprising mannitol or boric acid as a tonicity agent than from the other aqueous compositions. The result suggested that it is not preferable to add mannitol or boric acid as a tonicity agent to an aqueous composition comprising atropine and hydroxyethyl cellulose from the viewpoint of the stability of atropine.

In addition, the above results of Viscosity Determination Test 1 and Stability Test 1 suggested that it is preferable to add glycerin as a tonicity agent to an aqueous composition comprising atropine and hydroxyethyl cellulose to inhibit the decrease of viscosity over time and maintain the stability of atropine.

Test 6

(Viscosity Determination Test 2)

The effect of a tonicity agent to the viscosity of an aqueous composition comprising atropine and a water-soluble polymer was studied.

(Sample Preparation Method)

(Examples 23 to 28)

Aqueous compositions in Examples 23 to 28 were prepared as in Example 1 in accordance with the formulation shown in Table 7. Among those, Examples 26 to 28 are not in accordance with the present invention. Each prepared

sample (5 mL) was put into a polyethylene eyedrop container, an inside plug was attached to the bottle mouth, and the container was sealed-up with a cap. The containers were stored under dark at 60°C for 4 weeks.

| Table 7 (Unit: g/100 ml) | | | | | | |
|---|---|---|---|---|---|---|
| Example | 23 | 24 | 25 | 26 | 27 | 28 |
| Atropine sulfate hydrate | 0.01 | 0.01 | 0.01 | 0.01 | 0.01 | 0.01 |
| HPMC | 0.6 | 0.6 | 0.6 | - | - | - |
| CVP | - | - | - | 0.14 | 0.14 | 0.14 |
| Sodium dihydrogen phosphate | 0.1 | 0.1 | 0.1 | 0.1 | 0.1 | 0.1 |
| Concentrated glycerin | - | 2.4 | - | - | 2.4 | - |
| Mannitol | - | - | 5 | - | - | 5 |
| pH adjusting agent | q.s. | q.s. | q.s. | q.s. | q.s. | q.s. |
| pH | 5 | 5 | 5 | 5 | 5 | 5 |

(Test Method)

(Viscosity Determination Test 2)

According to "Method II, Viscosity measurement by rotational viscometer" in the Japanese Pharmacopoeia 16th edition, each viscosity of the freshly-prepared aqueous compositions and the aqueous compositions stored for 1, 2 and 4 weeks after the productions was measured with a cone-flat plate-type rotational viscometer. The measuring conditions are shown below.

- Instrument: Rotary rheometer (Kinexus pro+)
- Rotating speed (S-1): 50/sec
- Measurement temperature: 25°C

(Test Results)

The results of Examples 23 to 25 in the viscosity determination test are shown in Figure 3. The results of Examples 26 to 28 in the viscosity determination test are shown in Figure 4.

(Discussion)

(Viscosity Determination Test 2)

As shown in Figure 3, the viscosity of the aqueous composition comprising atropine and hydroxypropyl methylcellulose, but no tonicity agent (Example 23 which corresponds to "without" in Figure 3) decreased over time. On the other hand, the viscosity of the aqueous composition further comprising glycerin or mannitol as a tonicity agent (Example 24 or 25 which corresponds to "Glycerin" or "Mannitol" in Figure 3) was maintained, i.e., the decrease of viscosity over time was inhibited.

In addition, as shown in Figure 4, the viscosity of the aqueous composition comprising atropine and carboxyvinyl polymer, but no tonicity agent (Example 26 which corresponds to "without" in Figure 4) decreased over time. On the other hand, the viscosity of the aqueous composition further comprising glycerin or mannitol as a tonicity agent (Example 27 or 28 which corresponds to "Glycerin" or "Mannitol" in Figure 4) was maintained, i.e., the decrease of viscosity over time was inhibited.

Test 7

The effects of a water-soluble polymer to the actions for inhibiting the elongation of eye axial length and improving the refractive error were studied with myopia mouse models.

(Sample Preparation Method)

(Examples A to D)

Aqueous compositions in Examples A to D were prepared as in Example 1 in accordance with the formulation shown in Table 8. Among those, Examples A to C are not in accordance with the present invention.

Table 8 (Unit: g/100 ml)

| Example | A | B | C | D |
|---|---|---|---|---|
| Atropine sulfate hydrate | - | - | 0.01 | 0.01 |
| Hydroxyethyl cellulose | - | 0.45 | - | 0.45 |
| Sodium dihydrogen phosphate | 0.1 | 0.1 | 0.1 | 0.1 |
| Concentrated glycerin | 2.4 | 2.4 | 2.4 | 2.4 |
| pH adjusting agent | q.s. | q.s. | q.s. | q.s. |
| pH | 4.5 | 4.5 | 4.5 | 4.5 |

(Test Method)

Murine model of experimental myopia: Spectacle lens-induced myopia model was established by placing -10D lens on the right eye of the mice (C57BL/6J), which served as the experimental eye, at post-natal day 24. Briefly, a -10D lens (PMMA spectacle lens in blue tint, radius of outer curvature 8.5 mm, inner curvature 8 mm, lens thickness 0.5 mm) was glued to an annulus (with 8 mm base curve) of Velcro. This mating piece was then attached to the Velcro that had been glued to the hair around the right experimental eye using a cyanoacrylate. Through this set up, we made sure that an air gap of 1.5 mm existed between the back part of the lens and the anterior surface of the cornea.

Ocular biometry methods: Ocular biometry such as axial length and refractive error measurements were done using in vivo Optical Low Coherence Interferometry (OLCI-AcMaster) and automated eccentric photorefractor respectively. The axial length was measured at post-natal days 38 and 66, whereas the animal eyes were refracted at days 52 and 66.

Drug treatment: Atropine sulphate (at 0.01 % concentration) with and without hydroxyethyl cellulose were administered once a day at post-natal day 39 until day 66 in the spectacle lens-induced myopia model. 7 μL of each drug was administered topically to the right eye in dim red light each day.

(Test Results)

(1) Action for inhibiting the elongation of eye axial length

The results in Examples B to D are shown in Table 9.

The ratio for inhibiting the elongation of eye axial length with each Example was calculated by the following equation:

Difference of eye axial lengths (mm) = [eye axial length on day 66] - [eye axial length on day 38]

Ratio (%) for inhibiting the elongation of eye axial length with Example B =

$$\left(1-\frac{[\text{Difference of eye axial lengths in Example B administration group}]}{[\text{Difference of eye axial lengths in Example A administration group}]}\right)\times 100$$

Ratio (%) for inhibiting the elongation of eye axial length with Example C =

$$\left(1-\frac{[\text{Difference of eye axial lengths in Example C administration group}]}{[\text{Difference of eye axial lengths in Example A administration group}]}\right)\times 100$$

Ratio (%) for inhibiting the elongation of eye axial length with Example D =

$$\left(1-\frac{[\text{Difference of eye axial lengths in Example D administration group}]}{[\text{Difference of eye axial lengths in Example A administration group}]}\right)\times 100$$

| Table 9 | |
|---|---|
| Example | Ratio (%) for inhibiting the elongation of eye axial length |
| B | 0 |
| C | 5.81 |
| D | 24.65 |

(2) Action for improving the refractive error

The results in Examples A to D are shown in Table 10.

Change in refractive error (diopters) = [degree of refraction (diopters) on day 66] - [degree of refraction (diopters) on day 52]

| Table 10 | |
|---|---|
| Example | Change in refractive error (diopters) |
| A | -1.9 |
| B | -2.7 |
| C | -0.6 |
| D | +0.7 |

(Discussion)

The aqueous composition comprising the water-soluble polymer, but no atropine (Example B) had no action for inhibiting the elongation of eye axial length. On the other hand, it has been found that the aqueous composition comprising atropine and further the water-soluble polymer (Example D) has more potent action for inhibiting the elongation of eye axial length than the aqueous composition comprising atropine, but no water-soluble polymer (Example C).

In addition, as is the case with the action for inhibiting the elongation of eye axial length, it has been found that that the aqueous composition comprising atropine and further the water-soluble polymer (Example D) has more potent action for improving the refractive error than the aqueous composition comprising atropine, but no water-soluble polymer (Example C).

As is apparent from the results of Examples 1 and 3 in Tables 1 and 2, it is shown that the addition of a water-soluble polymer cannot create an adverse impact on the mydriatic action of atropine. Thus, an aqueous composition comprising atropine and a water-soluble polymer is expected to become an agent for inhibiting myopia progression which has a lower mydriatic action.

Preparation Examples

Medications of the present invention will be more specifically described by way of preparation examples; however, it should be noted that the present invention will not be limited only to those preparation examples.

Formulation Example 1: Eye Drop (0.01% (w/v))

| In 100 ml, | |
|---|---|
| Atropine sulfate hydrate | 0.01 |
| Hydroxyethyl cellulose | 0.32 g |
| Sodium dihydrogen phosphate | 0.1 g |
| Concentrated glycerin | 2.4 g |
| pH adjusting agent | q.s. |
| Sterile purified water | q.s. |

To sterile purified water are added atropine sulfate hydrate and the other ingredients as listed above. These ingredients are mixed well to prepare the above-described eye drop.

Formulation Example 2: Eye Drop (0.004% (w/v))

| In 100 ml, | |
|---|---|
| Atropine sulfate hydrate | 0.004 g |
| Hydroxyethyl cellulose | 0.32 g |
| Sodium dihydrogen phosphate | 0.1 g |
| Concentrated glycerin | 2.4 g |
| pH adjusting agent | q.s. |
| Sterile purified water | q.s. |

To sterile purified water are added atropine sulfate hydrate and the other ingredients as listed above. These ingredients are mixed well to prepare the above-described eye drop.

It has been shown that an aqueous composition comprising 0.001 - 0.1 % (w/v) atropine or a salt thereof, a water-soluble polymer, and buffer (I), which is at a pH range of 6 or lower, wherein the buffer (I) is at least one selected from the group consisting of a phosphate buffer and an aminocarboxylate buffer, wherein the water-soluble polymer is at least one selected from the group consisting of hydroxyethyl cellulose and hydroxypropyl methylcellulose, has a potent action for inhibiting the elongation of eye axial length and improving the refractive error without exacerbating the mydriatic action of atropine. Additionally, it has been also shown that the above aqueous composition, but which comprises no benzalkonium chloride or a limited amount of benzalkonium chloride, has a lower mydriatic action. Furthermore, it has been also shown that, in an aqueous composition comprising atropine or a salt thereof and a water-soluble polymer which is at a pH range of 6 or lower, the addition of a nonionic tonicity agent can make it possible to inhibit the debasement over time of the viscosity given by the water-soluble polymer and additionally maintain the stability of atropine or a salt thereof. The present aqueous composition is expected to inhibit or prevent the progression of myopia and lead to a lesser degree of mydriasis, and lesser loss of accommodation so as to be optimal in terms of quality of life.

## Claims

1. An aqueous composition comprising 0.001 - 0.1 % (w/v) atropine or a salt thereof, a water-soluble polymer, and buffer (I), which is at a pH range of 6 or lower, wherein the buffer (I) is at least one selected from the group consisting of a phosphate buffer and an aminocarboxylate buffer, wherein the water-soluble polymer is a cellulose derivative, and wherein the cellulose derivative is at least one selected from the group consisting of hydroxyethyl cellulose and hydroxypropyl methylcellulose.

2. The aqueous composition of claim 1, wherein the buffer (I) is a phosphate buffer.

3. The aqueous composition according to claim 1 or 2, further comprising a citrate buffer as buffer (II).

4. The aqueous composition according to any one of claims 1 to 3, which comprises less than 50 ppm benzalkonium chloride.

5. The aqueous composition according to any one of claims 1 to 4, further comprising a nonionic tonicity agent, wherein the nonionic tonicity agent is at least one selected from the group consisting of glycerin, mannitol, propylene glycol, polyethylene glycol, glucose, sorbitol, xylitol and trehalose.

6. The aqueous composition of claim 5, wherein the nonionic tonicity agent is at least one compound selected from the group consisting of glycerin and mannitol.

7. The aqueous composition according to any one of claims 3 to 6, wherein the concentration of citrate buffer is 0.001 - 1.0 % (w/v), preferably 0.01 - 0.05 % (w/v).

8. The aqueous composition according to any one of claims 1 to 7, wherein the concentration of the water-soluble polymer is 0.01 - 5 % (w/v).

9. The aqueous composition according to any one of claims 5 to 8, wherein the concentration of the nonionic tonicity agent is 0.01 to 10 % (w/v).

10. The aqueous composition according to any one of claims 1 to 9, wherein the concentration of the atropine of a salt thereof is 0.001 to 0.025% (w/v), preferably 0.001 to 0.01% (w/v).

11. The aqueous composition according to any one of claims 1 to 10, wherein the atropine or a salt thereof is atropine sulfate or a hydrate thereof.

12. The aqueous composition according to any one of claims 1 to 11 for use in inhibiting and/or preventing progression of myopia.

## Patentansprüche

1. Wässrige Zusammensetzung, die 0,001 - 0,1 % (w/v) Atropin oder ein Salz davon, ein wasserlösliches Polymer und Puffer (I) umfasst, welche in einem pH-Bereich von 6 oder niedriger vorliegt, wobei der Puffer (I) mindestens einer ist, ausgewählt aus der Gruppe, bestehend aus einem Phosphatpuffer und einem Aminocarboxylatpuffer, wobei das wasserlösliche Polymer ein Cellulosederivat ist und wobei das Cellulosederivat mindestens eines ist, ausgewählt aus der Gruppe, bestehend aus HydroxyethylCellulose und Hydroxypropyl-Methylcellulose.

2. Wässrige Zusammensetzung nach Anspruch 1, wobei der Puffer (I) ein Phosphatpuffer ist.

3. Wässrige Zusammensetzung nach Anspruch 1 oder 2, weiter umfassend einen Citratpuffer als Puffer (II).

4. Wässrige Zusammensetzung nach einem der Ansprüche 1 bis 3, welche weniger als 50 ppm Benzalkoniumchlorid umfasst.

5. Wässrige Zusammensetzung nach einem der Ansprüche 1 bis 4, weiter umfassend ein nichtionisches Tonizitätsmittel, wobei das nichtionische Tonizitätsmittel mindestens eines ist, ausgewählt aus der Gruppe, bestehend aus Glycerin, Mannitol, Propylenglycol, Polyethylenglycol, Glucose, Sorbitol, Xylitol und Trehalose.

6. Wässrige Zusammensetzung nach Anspruch 5, wobei das nichtionische Tonizitätsmittel mindestens eine Verbindung ist, ausgewählt aus der Gruppe, bestehend aus Glycerin und Mannitol.

7. Wässrige Zusammensetzung nach einem der Ansprüche 3 bis 6, wobei die Konzentration des Citratpuffers 0,001 - 1,0 % (w/v), bevorzugt 0,01 - 0,05 % (w/v), beträgt.

8. Wässrige Zusammensetzung nach einem der Ansprüche 1 bis 7, wobei die Konzentration des wasserlöslichen Polymers 0,01 - 5 % (w/v) beträgt.

9. Wässrige Zusammensetzung nach einem der Ansprüche 5 bis 8, wobei die Konzentration des nichtionischen

Tonizitätsmittels 0,01 bis 10 % (w/v) beträgt.

10. Wässrige Zusammensetzung nach einem der Ansprüche 1 bis 9, wobei die Konzentration des Atropins oder eines Salzes davon 0,001 bis 0,025 % (w/v), bevorzugt 0,001 bis 0,01 % (w/v), beträgt.

11. Wässrige Zusammensetzung nach einem der Ansprüche 1 bis 10, wobei das Atropin oder ein Salz davon Atropinsulfat oder ein Hydrat davon ist.

12. Wässrige Zusammensetzung nach einem der Ansprüche 1 bis 11 zur Verwendung zum Hemmen und/oder Verhindern des Fortschreitens von Kurzsichtigkeit.

**Revendications**

1. Composition aqueuse comprenant 0,001 à 0,1 % (p/v) d'atropine ou d'un sel de celle-ci, un polymère hydrosoluble et un tampon (I), qui est à une plage de pH de 6 ou inférieure, dans laquelle le tampon (I) est au moins un sélectionné parmi le groupe constitué d'un tampon de phosphate et d'un tampon d'aminocarboxylate, dans laquelle le polymère hydrosoluble est un dérivé de cellulose, et dans laquelle le dérivé de cellulose est au moins un sélectionné parmi le groupe constitué d'hydroxyéthylcellulose et d'hydroxypropylméthylcellulose.

2. Composition aqueuse selon la revendication 1, dans laquelle le tampon (I) est un tampon de phosphate.

3. Composition aqueuse selon la revendication 1 ou 2, comprenant en outre un tampon de citrate en tant que tampon (II).

4. Composition aqueuse selon l'une quelconque des revendications 1 à 3, qui comprend moins de 50 ppm de chlorure de benzalkonium.

5. Composition aqueuse selon l'une quelconque des revendications 1 à 4, comprenant en outre un agent de tonicité non ionique, dans laquelle l'agent de tonicité non ionique est au moins un sélectionné parmi le groupe constitué de la glycérine, du mannitol, du propylèneglycol, du polyéthylèneglycol, du glucose, du sorbitol, du xylitol et du tréhalose.

6. Composition aqueuse selon la revendication 5, dans laquelle l'agent de tonicité non ionique est au moins un composé sélectionné parmi le groupe constitué de la glycérine et du mannitol.

7. Composition aqueuse selon l'une quelconque des revendications 3 à 6, dans laquelle la concentration du tampon de citrate est de 0,001 à 1,0 % (p/v), de préférence 0,01 à 0,05 % (p/v).

8. Composition aqueuse selon l'une quelconque des revendications 1 à 7, dans laquelle la concentration du polymère hydrosoluble est de 0,01 à 5 % (p/v).

9. Composition aqueuse selon l'une quelconque des revendications 5 à 8, dans laquelle la concentration de l'agent de tonicité non ionique est de 0,01 à 10 % (p/v).

10. Composition aqueuse selon l'une quelconque des revendications 1 à 9, dans laquelle la concentration de l'atropine ou d'un sel de celle-ci est de 0,001 à 0,025 % (p/v), de préférence 0,001 à 0,01 % (p/v).

11. Composition aqueuse selon l'une quelconque des revendications 1 à 10, dans laquelle l'atropine ou un sel de celle-ci est le sulfate d'atropine ou un hydrate de celui-ci.

12. Composition aqueuse selon l'une quelconque des revendications 1 à 11 destinée à être utilisée dans l'inhibition et/ou la prévention de la progression de la myopie.

[Fig. 1]

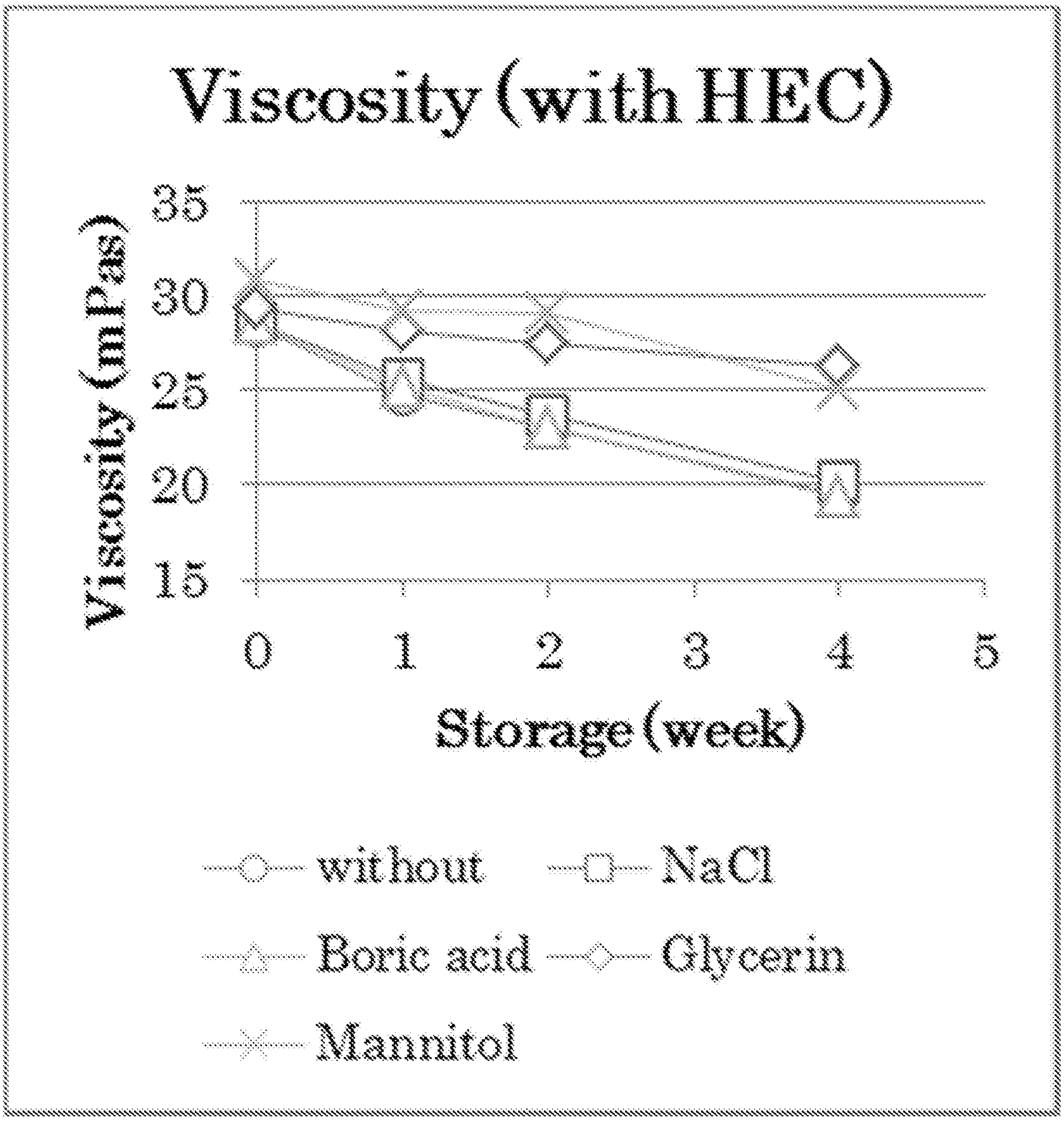
Viscosity (with HEC)
Viscosity (mPas)
35
30
25
20
15
0
1
2
3
4
5
Storage (week)
without
NaCl
Boric acid
Glycerin
Mannitol

[Fig. 2]

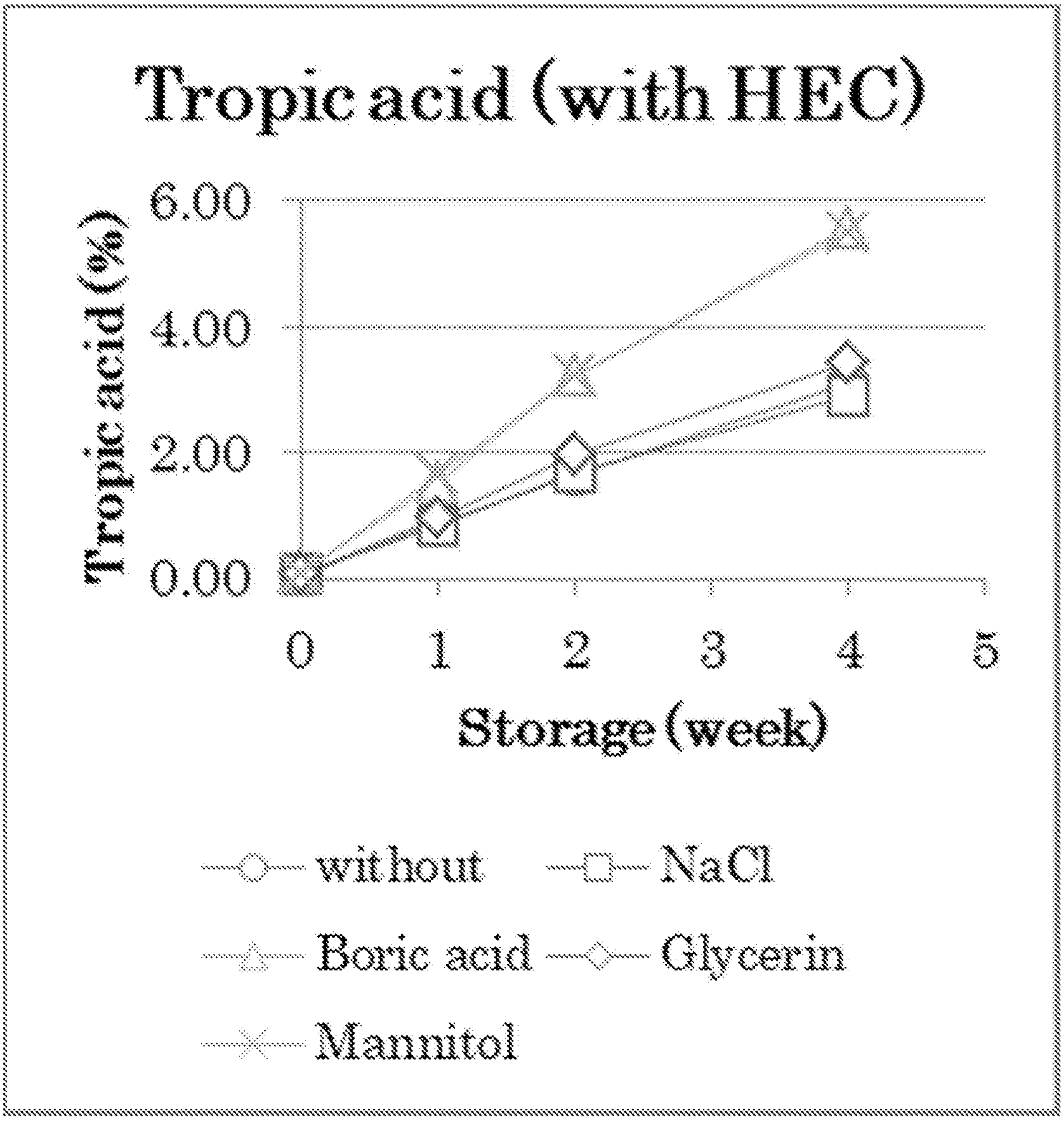
Tropic acid (with HEC)
Tropic acid (%)
6.00
4.00
2.00
0.00
0
1
2
3
4
5
Storage (week)
without
NaCl
Boric acid
Glycerin
Mannitol

[Fig. 3]

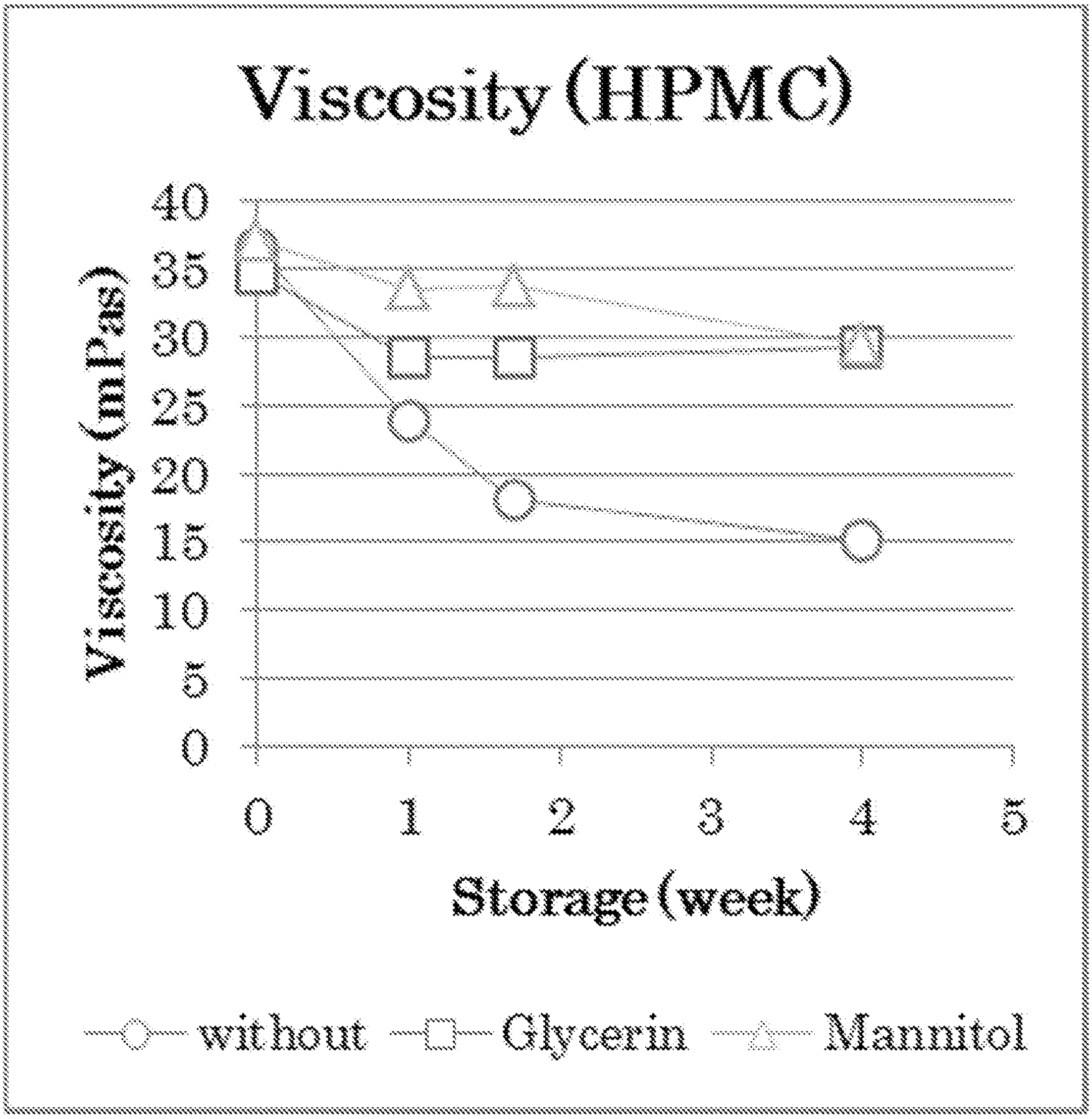
Viscosity (HPMC)
Viscosity (mPas)
40
35
30
25
20
15
10
5
0
0
1
2
3
4
5
Storage (week)
without
Glycerin
Mannitol

[Fig. 4]

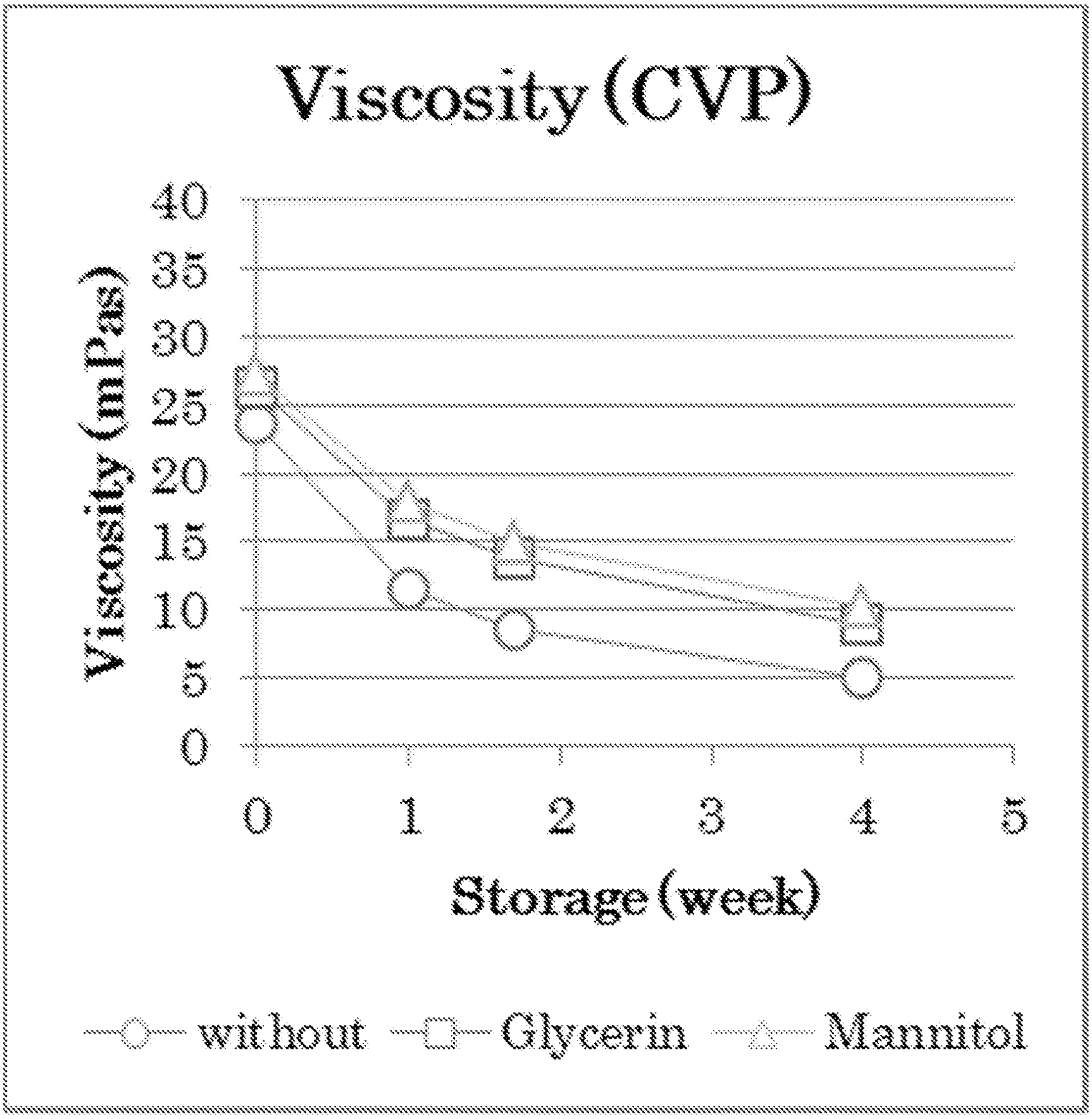
Viscosity (CVP)
Viscosity (mPas)
40
35
30
25
20
15
10
5
0
0
1
2
3
4
5
Storage (week)
without
Glycerin
Mannitol

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012161655 A1
- US 2016009705 A1
- WO 2015200361 A1